# EUROPEAN PATENT APPLICATION

(11) **EP 4 361 288 A1**
(43) Date of publication of application: **01.05.2024**
(21) Application number: 22383026.6
(22) Date of filing: 25.10.2022
(51) Int. Cl.: C12Q 1/6886

(54) **PERSONALIZED IMMUNOTHERAPY IN RENAL CARCINOMA**

(71) Applicant: Fundación Instituto de Investigación Sanitaria de Santiago de Compostela (FIDIS), Santiago de Compostela (ES); Servizo Galego de Saude, 15703 Santiago de Compostela (ES)
(72) Inventor: RUIZ BAÑOBRE, Juan, 15706 Santiago de Compostela (ES); LÓPEZ LÓPEZ, Rafael, 15706 Santiago de Compostela (ES); BETANCOR RIVERO, Yoel Zacarías, 15706 Santiago de Compostela (ES)
(74) Representative: Hoffmann Eitle

(57) **Abstract**

The present invention refers to an *in vitro* method for predicting the prognosis of renal carcinoma, for predicting the benefit of patients suffering from renal carcinoma from a treatment with antibody-based immunotherapy and/or for deciding or recommending whether to treat patients suffering from renal carcinoma with antibody-based immunotherapy.

## Description

### FIELD OF THE INVENTION

The present invention refers to the medical field. Particularly, the present invention refers to an *in vitro* method for predicting the prognosis of renal carcinoma, for predicting the benefit of patients suffering from renal carcinoma from a treatment with antibody-based immunotherapy and/or for deciding or recommending whether to treat patients suffering from renal carcinoma with antibody-based immunotherapy.

### STATE OF THE ART

Renal cell carcinoma (RCC), also known as kidney cancer or renal cancer, is one of the most frequently diagnosed type of cancer (sixth in males and ninth in females in the USA in 2022). RCC is approximately twice as prevalent in males compared to females, and the peak of prevalence by age is in the 75-79 range.

All RCC originate from the epithelial cells of the proximal tubule of the nephron, but despite the common origin, RCC can manifest in different subtypes with particular histological, molecular and clinical features. Of the histological subtypes, clear cell RCC (ccRCC) is by far the most commonly diagnosed, representing about 75% of the cases. Other less common types are papillary type 1 (15%), chromophobe (5%) and clear cell papillary RCC (1-4%).

The comprehensive molecular analyses of tumour samples have revealed that the different subtypes of RCC present distinct mutational, methylation and expression profiles, and these differences may lead to the discovery of possible therapeutic targets for the development of subtype-specific treatments. For example, ccRCC commonly present an inactivation of the gene VHL, which causes an overexpression of the pro-angiogenic factor VEGF and thus an increased vascularity in the tumour. In addition, ccRCC commonly presents loss of tumour suppressor genes associated with the short arm of chromosome 3 (PBRM1, 40%; BAP1, 10%; and SETD2, 10%). Type-1 papillary RCC, on the other hand, presents mutations in MET (13% of cases) that lead to an increased proliferation rate and resistance to apoptosis.

One important characteristic of ccRCC tumours is the presence of a considerable immune infiltration and expression of Programmed cell Death 1 (PD-1) and Cytotoxic T-lymphocyte-Associated protein 4 (CTLA-4), as detected in pan-cancer analyses. PD-1, Programmed Death Ligand 1 (PD-L1) and CTLA-4 are immune checkpoint proteins, which mediate the inactivation of T cells. This mechanism exists to avoid the hyperactivation of the immune system and therefore prevents autoimmune reactions against the individual's own tissues. For cancer cells, these molecules represent a highly advantageous way to avoid the normal anti-tumoural response of the immune system. Based on these facts, together with previous evidence of response of RCC to cytokine-based therapies, ccRCC has been considered a great candidate for the use of immune checkpoint inhibitors (ICI): monoclonal antibodies that block PD-1 (or its ligand PD-L1) or CTLA-4 receptors.

Over the last decade, various ICIs have emerged for the treatment of unresectable advanced or metastatic renal cell carcinoma (mRCC). In the first line setting, today there are available several ICI-based regimens, which have been approved by US Food and Drug Administration and the European Medicines Agency after demonstrating significant overall survival improvements in randomised, phase 3 clinical trials: pembrolizumab with either axitinib or lenvatinib (both tyrosine kinase inhibitors), nivolumab with cabozantinib (tyrosine kinase inhibitor), and nivolumab with ipilimumab (this combination only for those patients with intermediate or poor-risk mRCC). In the second line setting, nivolumab is approved as a standard treatment option for mRCC patients following prior antiangiogenic therapy. This approval was based on the positive results of the CheckMate 025 (CM-025) trial, a randomised open-label phase 3 study where patients treated with nivolumab achieved significant survival and response improvements compared to those treated with everolimus. On the other hand, in the adjuvant setting, pembrolizumab is indicated for the treatment of adults with RCC at increased risk of recurrence following nephrectomy or following nephrectomy and resection of metastatic lesions.

Since the popularisation of next generation sequencing and the birth of the "Transcriptomics" field, there has been a growing effort in the study of gene expression profiles of tumours and their association with response to cancer treatments, all in order to identify prognostic and/or predictive biomarkers to guide cancer patient management. Finding these biomarkers would be highly beneficial not only for improving survival in cancer patients, but also for sparing them from unnecessary toxicity and reducing the economic burden of expensive treatments.

Gene expression-based biomarkers for RCC patients are a current subject of research, for example, analyses of the IMmotion150 phase 2 clinical trial comparing atezolizumab (anti-PD-L1) alone or in combination with bevacizumab (anti-VEGF) against sunitinib (tyrosine kinase receptor inhibitor) revealed gene expression signatures strongly associated with differences in progression free survival (PFS) between the arms of the study. In addition, new gene expression signatures have been studied that were correlated with a higher PFS in patients from the phase 3 JAVELIN Renal 101 trial treated with avelumab (anti-PD-L1) plus axitinib (tyrosine kinase inhibitor) compared to sunitinib.

According to the prior art, there is an unmet medical need of finding reliable tools for predicting the prognosis of renal carcinoma, for predicting the benefit of patients suffering from renal carcinoma from a treatment with antibody-based immunotherapy and/or for deciding or recommending whether to treat patients suffering from renal carcinoma with antibody-based immunotherapy. The present invention is focused on solving this problem and a new and innovative strategy is herein provided.

### DESCRIPTION OF THE INVENTION

### Brief description of the invention

The present invention refers to an *in vitro* method for predicting the prognosis of renal carcinoma, for predicting the benefit of patients suffering from renal carcinoma from a treatment with antibody-based immunotherapy and/or for deciding or recommending whether to treat patients suffering from renal carcinoma with antibody-based immunotherapy.

The inventors of the present invention, knowing that, unfortunately, not all patients suffering from renal carcinoma benefit from immunotherapy, and that there is indeed a lack of reliable prognostic and predictive biomarkers to guide treatment decisions in the clinical setting, have focused their efforts on identifying not only prognostic biomarkers but also biomarkers able to predict the benefit to immunotherapy, particularly anti-PD-1 antibodies.

As proof of concept, seven genes MYO9B (HGNC: 7609), AUP1 (HGNC: 891), HMGA1 (HGNC: 5010), USF1 (HGNC: 12593), NUP62 (HGNC: 8066), ARHGAP42 (HGNC: 26545) or MOCS1 (HGNC: 7190) were found with prognostic significance for overall survival in nivolumab-treated patients, which in addition, were significantly correlated with either disease control, response, or both. Then, the prognostic significance of these selected genes was validated in The Cancer Genome Atlas Kidney Renal Clear Cell Carcinoma cohort. Finally, the interaction of the treatment and the expression level of the genes was tested and proved to be statistically significant for overall survival for the seven genes.

So, in conclusion, the inventors of the present invention were able to identify and validate the prognostic and predictive significance for overall survival of the above cited seven genes in patients diagnosed with advanced or metastatic renal carcinoma treated, as a proof of concept, with nivolumab.

So, the first embodiment of the present invention refers to an *in vitro* method for predicting the prognosis of renal carcinoma in a patient which comprises: a) Assessing the level of expression of at least one of the following genes included in the group consisting of: MYO9B, AUP1, HMGA1, USF1, NUP62, ARHGAP42 and/or MOCS1 (or any combination thereof comprising of between 2 and 7 genes), in a biological sample, such as tumor sample, obtained from the patient, b) wherein the identification of a higher level of expression of MYO9B, AUP1, HMGA1, USF1 and/or NUP62 (or any combination thereof comprising of between 2 and 5 genes) which respect to a pre-established threshold value is indicative of poor prognosis; or wherein the identification of a lower level of expression of MY09B, AUP1, HMGA1, USF1 and/or NUP62 (or any combination thereof comprising of between 2 and 5 genes) which respect to a pre-established threshold value is indicative of good prognosis; or c) wherein the identification of a higher level of expression of ARHGAP42 and/or MOCS1 which respect to a pre-established threshold value is indicative of good prognosis; or wherein the identification of a lower level of expression of ARHGAP42 and/or MOCS1 which respect to a pre-established threshold value is indicative of poor prognosis.

The second embodiment of the present invention refers to an *in vitro* method for predicting the benefit of patients suffering from renal carcinoma from a treatment with antibody-based immunotherapy which comprises: a) assessing the level of expression of at least one of the following genes included in the group consisting of: MYO9B, AUP1, HMGA1, USF1, NUP62, ARHGAP42 and/or MOCS1 (or any combination thereof comprising of between 2 and 7 genes), in a biological sample obtained from the patient, b) wherein the identification of a lower level of expression of MY09B, AUP1, HMGA1, ARHGAP42 and/or MOCS1 (or any combination thereof comprising of between 2 and 5 genes) which respect to a pre-established threshold value is indicative that the patient is benefitting from the therapy; or c) wherein the identification of a higher level of expression of USF1 and/or NUP62 which respect to a pre-established threshold value is indicative that the patient is benefitting from the therapy.

The third embodiment of the present invention refers to an *in vitro* method for deciding or recommending whether to treat patients suffering from renal carcinoma with antibody-based immunotherapy which comprises: a) assessing the level of expression of at least one of the following genes included in the group consisting of: MYO9B, AUP1, HMGA1, USF1, NUP62, ARHGAP42 and/or MOCS1 (or any combination thereof comprising of between 2 and 7 genes) in a biological sample obtained from the patient, b) wherein if a lower level of expression of MYO9B, AUP1, HMGA1, ARHGAP42 and/or MOCS1 (or any combination thereof comprising of between 2 and 5 genes) is identified which respect to a pre-established threshold value a treatment with antibody-based immunotherapy is recommended; or c) wherein if a higher level of expression of USF1 and/or NUP62 is identified which respect to a pre-established threshold value a treatment with antibody-based immunotherapy is recommended.

In a preferred embodiment, the antibody-based immunotherapy comprises an anti-PD-1 or an anti-PD-L1 antibody.

In a preferred embodiment, the antibody-based immunotherapy comprises an anti-PD-1 or an anti-PD-L1 antibody selected from: nivolumab, pembrolizumab, dostarlimab, sintilimab, cemiplimab, atezolizumab, durvalumab or avelumab.

The fourth embodiment of the present invention refers to the *in vitro* use of at least one of the following genes included in the group consisting of: MYO9B, AUP1, HMGA1, USF1, NUP62, ARHGAP42 and/or MOCS1 (or any combination thereof comprising of between 2 and 7 genes), for predicting the prognosis of renal carcinoma or for predicting the benefit of patients suffering from renal carcinoma from a treatment with antibody-based immunotherapy.

In a preferred embodiment, the present invention refers to the *in vitro* use of at least one of the following genes included in the group consisting of: MYO9B, AUP1, HMGA1, USF1, NUP62, ARHGAP42 and/or MOCS1 (or any combination thereof comprising of between 2 and 7 genes) for predicting the benefit of patients suffering from renal carcinoma from a treatment with antibody-based immunotherapy comprising an anti-PD-1 antibody.

In a preferred embodiment, the present invention refers to the *in vitro* use of at least one of the following genes included in the group consisting of: MYO9B, AUP1, HMGA1, USF1, NUP62, ARHGAP42 and/or MOCS1 (or any combination thereof comprising of between 2 and 7 genes) for predicting the benefit of patients suffering from renal carcinoma from a treatment with antibody-based immunotherapy comprising an anti-PD-1 or an anti-PD-Ll antibody selected from: nivolumab, pembrolizumab, dostarlimab, sintilimab, cemiplimab, atezolizumab, durvalumab or avelumab.

The fifth embodiment of the present invention refer to a kit for performing any of the above methods which consist of reagents or tools to assess the level of expression of the genes: MYO9B, AUP1, HMGA1, USF1, NUP62, ARHGAP42 and/or MOCS1 (or any combination thereof comprising of between 2 and 7 genes).

The sixth embodiment of the present invention refers to anti-PD-1 or anti-PD-L1 antibody for use in the treatment of renal carcinoma in a patient, wherein the patient is characterized by a lower expression level of at least one of the following genes included in the group consisting of: MYO9B, AUP1, HMGA1, ARHGAP42 and/or MOCS1 (or any combination thereof comprising of between 2 and 5 genes); or by a higher level of expression level of at least one of the following genes included in the group consisting of: USF1 and/or NUP62, which respect to a pre-established threshold value.

In a preferred embodiment the present invention refers to anti-PD-1 or anti-PD-Ll antibody for use in the treatment of renal carcinoma in a patient wherein the patient is characterized by a lower expression level of at least one of the following genes included in the group consisting of: MYO9B, AUP1, HMGA1, ARHGAP42 and/or MOCS1 (or any combination thereof comprising of between 2 and 5 genes); or by a higher level of expression of at least one of the following genes included in the group consisting of: USF1 and/or, which respect to a pre-established threshold value, and wherein the method comprises the step of determining whether the patient has a lower expression level of at least one of the following genes included in the group consisting of: MYO9B, AUP1, HMGA1, ARHGAP42 and/or MOCS1 (or any combination thereof comprising of between 2 and 5 genes), or a higher level expression level of at least one of the following genes included in the group consisting of: USF1 and/or NUP62, which respect to a pre-established threshold value.

In a preferred embodiment, the anti-PD-1 or anti-PD-Ll antibody is selected from: nivolumab, pembrolizumab, dostarlimab, sintilimab, cemiplimab, atezolizumab, durvalumab or avelumab.

Alternatively, the present invention also refers to:
Method for detecting the level of expression of at least one of the following genes included in the group consisting of: MYO9B, AUP1, HMGA1, USF1, NUP62, ARHGAP42 and/or MOCS1 (or any combination thereof comprising of between 2 and 7 genes), previous to the prediction of the prognosis of renal carcinoma, the prediction of the benefit of patients suffering from renal carcinoma from a treatment with antibody-based immunotherapy, or previous to deciding or recommending whether to treat patients suffering from renal carcinoma with antibody-based immunotherapy, which comprises: a) contacting the biological sample with a reagent capable of specifically binding the genes and b) measuring the level of expression of the genes.

Method for analysing a biological sample previous to the prediction of the prognosis of renal carcinoma, the prediction of the benefit of patients suffering from renal carcinoma from a treatment with antibody-based immunotherapy, or previous to deciding or recommending whether to treat patients suffering from renal carcinoma with antibody-based immunotherapy, comprising analysing the biological sample to assess the level of expression of at least one of the following genes included in the group consisting of: MYO9B, AUP1, HMGA1, USF1, NUP62, ARHGAP42 and/or MOCS1 (or any combination thereof comprising of between 2 and 7 genes)

In a preferred embodiment, the present invention is a computer-implemented invention, wherein a processing unit (hardware) and a software are configured to:
- Receive the level of expression of the biomarkers,
- Process the level of expression of the biomarkers for finding substantial variations or deviations, and
- Provide an output through a terminal display.

Method for treating patients suffering from renal carcinoma, which comprises the administration of a therapeutically effective amount of treatment known in the prior art to treat renal carcinoma, the method comprising performing, as a first step, the methods of the invention describe above.

Method for treating renal carcinoma in a patient which comprises the administration of a therapeutically effective amount of an anti-PD-1 or anti-PD-Ll antibody, wherein the patient is characterized by a lower expression level of at least one of the following genes included in the group consisting of: MYO9B, AUP1, HMGA1, ARHGAP42 and/or MOCS1 (or any combination thereof comprising of between 2 and 5 genes); or by a higher level of expression level of at least one of the following genes included in the group consisting of: USF1 and/or NUP62, which respect to a pre-established threshold value.

Method for treating renal carcinoma in a patient which comprises the administration of a therapeutically effective amount of an anti-PD-1 or anti-PD-Ll antibody, wherein the patient is characterized by a lower expression level of at least one of the following genes included in the group consisting of: MYO9B, AUP1, HMGA1, ARHGAP42 and/or MOCS1 (or any combination thereof comprising of between 2 and 5 genes); or by a higher level of expression level of at least one of the following genes included in the group consisting of: USF1 and/or NUP62, which respect to a pre-established threshold value, and wherein the method comprises the step of determining whether the patient has a lower expression level of at least one of the following genes included in the group consisting of: MYO9B, AUP1, HMGA1, ARHGAP42 and/or MOCS1 (or any combination thereof comprising of between 2 and 5 genes), or a higher level expression level of at least one of the following genes included in the group consisting of: USF1 and/or NUP62, which respect to a pre-established threshold value.

For the purpose of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- By "effective dose or amount" is intended an amount that, when administered as described herein, brings about a measurable response in the host. The exact amount required will vary from subject to subject, depending on the age, and general condition of the subject, the severity with which the disease has been/is progressed, mode of administration, and the like. An appropriate "effective" amount in any individual case may be determined by one of ordinary skill in the art using routine experimentation, based upon the information provided herein.
- According to the present invention, a reference value can be a "pre-established threshold value" or a "cut-off" value. Typically, a "threshold value" or "cut-off value" can be determined experimentally, empirically, or theoretically. According to the present invention, the "pre-established threshold" value refers to a value previously determined in renal cell carcinoma patients. A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative).

### Description of the figures

**Figure 1****.** Distribution of disease control (a) and response (b) in the nivolumab-treated cohort categorised by the expression levels of the selected genes. P values were calculated with Chi-squared and Fisher's Exact test, respectively. Abbreviations: DC, "Disease Control"; R, "Response".
**Figure 2****.** Kaplan-Meier curves of the genes with a significant correlation with survival separated by expression level of the TCGA KIRC cohort. P values were calculated with Log-Rank tests.
**Figure 3****.** Kaplan-Meier curves for those genes with a significant correlation with overall survival in the TCGA KIRC stage IV subcohort. P values were calculated with Log-Rank tests.
**Figure 4****.** Kaplan-Meier curves of the CM-025 phase 3 clinical trial cohort separated by the expression level of each gene of interest and arm of treatment. P values were calculated with Log-Rank tests.

### Detailed description of the invention

The present invention is illustrated by means of the Examples set below without the intention of limiting its scope of protection.

### Example 1. Material and methods

### Example 1.1. Study design and patient population

This is a retrospective study of patients diagnosed with advanced or metastatic ccRCC treated with either nivolumab or everolimus in a clinical trial scenario or from TCGA KIRC. Patient data from three prospective clinical trials of the anti-PD-1 antibody nivolumab in advanced ccRCC were used in this study: CheckMate 009 (CM-009; NCT01358721), CheckMate 010 (CM-010; NCT01354431) and CheckMate 025 (CM-025; NCT01668784). Patients enrolled in CM-010 and CM-025 had advanced (metastatic) RCC with clear cell histology and had progressed on at least one prior systemic anti-angiogenic therapy (CM-009 had similar eligibility criteria, though a subset of n = 10 patients were treated with nivolumab as frontline therapy). CM-010 was a phase II dose-finding study of nivolumab, which found no dose-dependent correlation with PFS. CM-025 was a randomised phase III trial that demonstrated an improved overall survival with nivolumab over the mTOR inhibitor everolimus.

### Example 1.2. Data preprocessing

The expression matrices of CM-009, CM-010 and CM-025 cohorts provided by Braun *et al.* (Braun et al. 2020) [Braun, David A., Yue Hou, Ziad Bakouny, Miriam Ficial, Miriam Sant' Angelo, Juliet Forman, Petra Ross-Macdonald, et al. 2020. "Interplay of Somatic Alterations and Immune Infiltration Modulates Response to PD-1 Blockade in Advanced Clear Cell Renal Cell Carcinoma." Nature Medicine 26 (6, 6): 909-18], which were previously corrected for batch effect by the authors, were used for the analysis. For each cohort, the expression levels of all genes in Transcripts per Million (TPMs) were dichotomised into *high* and *low* using the optimal cut-off method implemented in the maxstat package in R. The same process was used for the Fragments Per Kilobase of transcript per Million mapped reads (FPKM) expression matrix from The Cancer Genome Atlas Kidney Renal Clear Cell Carcinoma (TCGA KIRC) cohort.

### Example 1.3. Identification of genes with prognostic significance for overall survival in the nivolumab-treated cohort

Cox Proportional Hazards (PH) models were generated using the dichotomised gene expression data of nivolumab-treated patients (n = 181) via the coxph() function from the package survival and RegParallel for automated parallelised computing. The P values of the two-sided Wald tests were corrected for False Discovery Rate (FDR) using the Bonferroni method. Genes with *Q* < 0.05 were selected (from now on as *selected genes*) for subsequent analyses. The PH assumption was tested for the selected genes using the cox.zph() function. Kaplan-Meier curves and Log-Rank tests were calculated using the survminer package.

### Example 1.4. Validation of the prognostic significance for overall survival of the selected genes in the TCGA KIRC cohort

For consistency, Cox PH models, Kaplan-Meier curves and Log-Rank tests were calculated using the same methods as with the nivolumab-treated cohort.

### Example 1.5. Tests of independence

Patients were grouped by their tumour response according to Response Evaluation Criteria in Solid Tumors guidelines version 1.1 in complete response (CR), partial response (PR), stable disease (SD), progressive disease (PD), disease control (CR+PR+SD) or no disease control (PD+NE); and response (CR+PR) or no response (SD+PD+NE). Patients who died before radiologic assessment were considered as not evaluable for response (NE).

Correlations between the expression level of the selected genes and the clinical response groups were tested using two-sided Chi-squared tests or Fisher's Exact tests when the n in a group was too small to perform an informative Chi-squared test. *P* values < 0.05 were considered statistically significant.

### Example 1.6. Logistic regression

Univariate logistic regressions were calculated using the glm() function form base R, specifying the family as binomial due to the categorical nature of the variables. The same groups from the tests of independence were used. *P* values < 0.05 were considered statistically significant.

### Example 1.7. Interaction tests

Cox PH models were constructed using the CM-025 cohort for each selected gene considering the interaction term between the gene expression category (high *vs* low) and therapy (nivolumab *vs* everolimus). The HR for each interaction with nivolumab and the associated P value was reported. The models for interaction's overall goodness-of-fit was measured using the Likelihood Ratio Test (LTR). *P* values < 0.05 were considered statistically significant.

### Example 2. Results

### Example 2.1. Identification of genes with prognostic significance for overall survival in the nivolumab-treated cohort

To detect genes that correlate with patient survival, we performed univariate Cox Proportional Hazard (Cox PH) regressions and Log-Rank tests in the nivolumab-treated cohort correcting for FDR by the Bonferroni method (**Table 1**). Log-Rank genes and Kaplan-Meier curves were calculated for genes with a Cox PH ratio's *Q* < 0.05.

**Table 1. Genes with a statistically significant prognostic value for overall survival in the nivolumab-treated cohort as observed in Cox PH univariate models.**

| **Gene** | **Coefficient (β)** | **Hazard Ratio (95% CI)** | **Q (Wald test)** |
|---|---|---|---|
| SPOCD1 | 1.143 | 3.137 (2.110 - 4.663) | 0.001 ^{∗} |
| MYO9B | 1.428 | 4.168 (2.489 - 6.981) | 0.003 ^{∗} |
| AUP1 | 1.002 | 2.724 (1.896 - 3.914) | 0.003 ^{∗} |
| HMGA1 | 1.005 | 2.732 (1.891 - 3.947) | 0.004 ^{∗} |
| HIST2H2BF | 1.184 | 3.268 (2.105 - 5.074) | 0.006 ^{∗} |
| USF1 | 1.146 | 3.146 (2.043 - 4.845) | 0.009 ^{∗} |
| NUP62 | 0.951 | 2.587 (1.804 - 3.711) | 0.010 ^{∗} |
| ARHGAP42 | -0.942 | 0.390 (0.271 - 0.561) | 0.017 ^{∗} |
| ADCY5 | -0.960 | 0.383 (0.264 - 0.555) | 0.017 ^{∗} |
| MOCS1 | -1.011 | 0.364 (0.245 - 0.540) | 0.022 ^{∗} |
| RP5-1107A17.2 | 1.082 | 2.950 (1.931 - 4.506) | 0.025 ^{∗} |
| PSMD10P2 | 1.271 | 3.563 (2.163 - 5.869) | 0.026 ^{∗} |
| CLIC4 | -1.211 | 0.298 (0.184 - 0.482) | 0.035 ^{∗} |
| CDH5 | -0.995 | 0.370 (0.249 - 0.550) | 0.039 ^{∗} |
| HMMR | 1.047 | 2.850 (1.873 - 4.336) | 0.044 ^{∗} |
| HSF2BP | 0.916 | 2.499 (1.729 - 3.613) | 0.048 ^{∗} |
| DIAPH3 | 1.012 | 2.752 (1.831 - 4.136) | 0.049 ^{∗} |

| | | | |
|---|---|---|---|
| Asterisks indicate statistical significance (*Q* < 0.05). | | | |

All selected genes had a significant effect on overall survival determined with the Log-Rank test. A high expression of genes SPOCD1, MYO9B, AUP1, HMGA1, HIST2H2BF, USF1, NUP62, RP5-1107A17.2, PSMD10P2, HMMR, HSF2BP and DIAPH3 was correlated with worse overall survival. For genes ARHGAP42, ADCY5, MOCS1, CLIC4 and CDH5 the opposite correlation was observed.

### Example. 2.2. Correlation between the expression level of the selected genes and disease control and response in the nivolumab-treated cohort

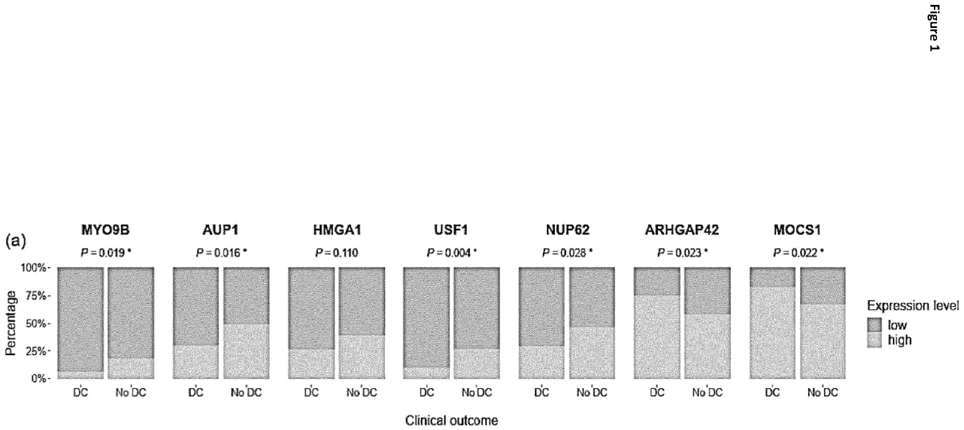

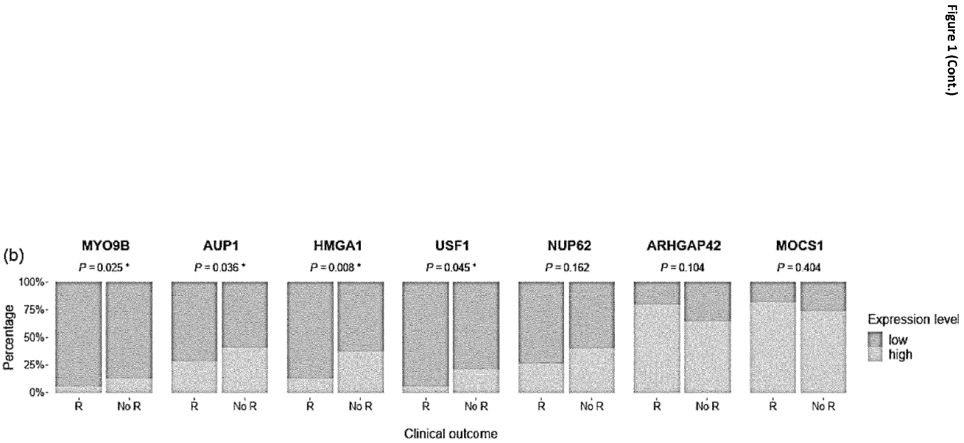

*Distribution statistics.* In order to test if the expression level of the selected genes was correlated with the distribution of the patients into the different clinical outcome categories, we performed Chi-squared tests for disease Control (DC) and Fisher's Exact Test for response. Of the 17 genes with a significant prognostic power for survival, only 7 showed significant correlation with the distribution of clinical outcomes (**Figure 1**).

*Logistic regressions.* Univariate logistic regressions were calculated to evaluate the predictive power of the expression level of each gene over the classification of the patients into DC or response **(Table 2)**. As with the independence test, 7 of the 17 prognostic genes showed a significant association with the clinical outcome based in their Odds Ratio (OR) and Wald test *P* value, either with DC (MY09B,AUP1, USF1, NUP62, ARHGAP42 and MOCS1) or response (HMGA1 and USF1). The results agree with the effect determined in the Cox PH and Kaplan-Meier models, with genes MYO9B to NUP62 having a negative correlation with DC and/or response (OR < 1), and ARHGAP42 and MOCS1 having a positive correlation with DC (OR > 1).

**Table 2. Univariate logistic regressions of the selected genes for the nivolumab-treated cohort.**

| | **Disease control** | | **Response** | |
|---|---|---|---|---|
| **Gene** | **OR (95% CI)** | **P (Wald test)** | **OR (95% CI)** | **P (Wald test)** |
| MYO9B | 0.28 (0.10 - 0.77) | 0.014 ^{∗} | 0.37 (0.08 - 1.68) | 0.199 |
| AUP1 | 0.45 (0.25 - 0.84) | 0.011 ^{∗} | 0.57 (0.26 - 1.23) | 0.153 |
| HMGA1 | 0.57 (0.30 - 1.07) | 0.080 | 0.25 (0.09 - 0.69) | 0.007 ^{∗} |
| USF1 | 0.29 (0.13 - 0.66) | 0.003 ^{∗} | 0.21 (0.05 - 0.93) | 0.039 ^{∗} |
| NUP62 | 0.48 (0.26 - 0.89) | 0.019 ^{∗} | 0.53 (0.24 - 1.17) | 0.116 |
| ARHGAP42 | 2.17 (1.15 - 4.09) | 0.016 ^{∗} | 2.17 (0.93 - 5.08) | 0.074 |
| MOCS1 | 2.36 (1.18 - 4.72) | 0.015 ^{∗} | 1.61 (0.66 - 3.96) | 0.299 |

| | | | | |
|---|---|---|---|---|
| OR: Odds Ratio. Asterisks indicate statistical significance (P < 0.05). | | | | |

After these results, we decided to perform further analyses only with the genes that had a significant correlation with either DC or response.

### Example. 2.3. Validation of the prognostic significance of the selected genes for overall survival in the TCGA KIRC cohort

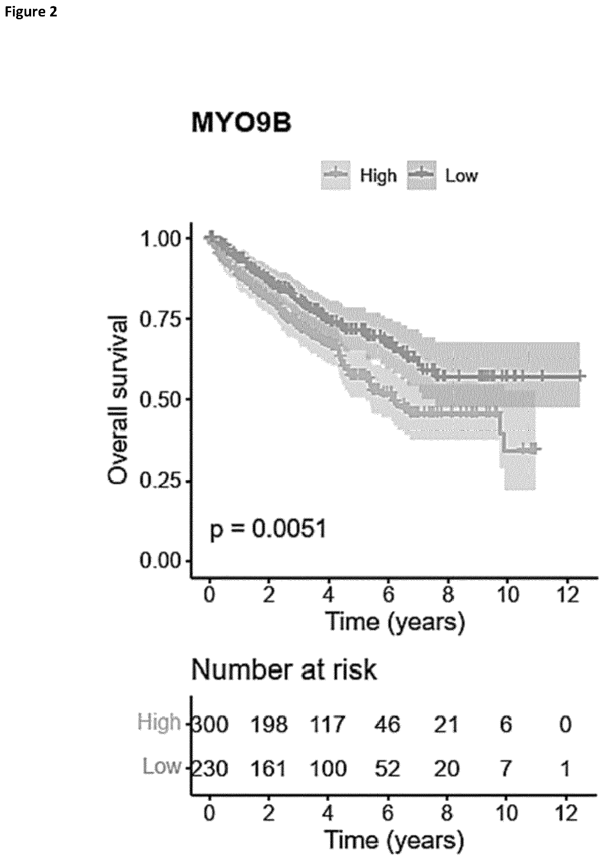

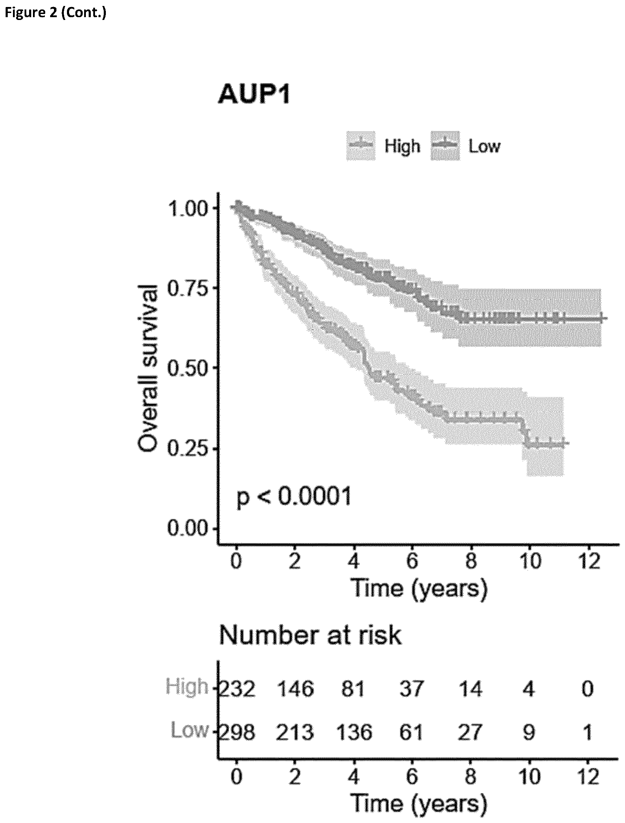

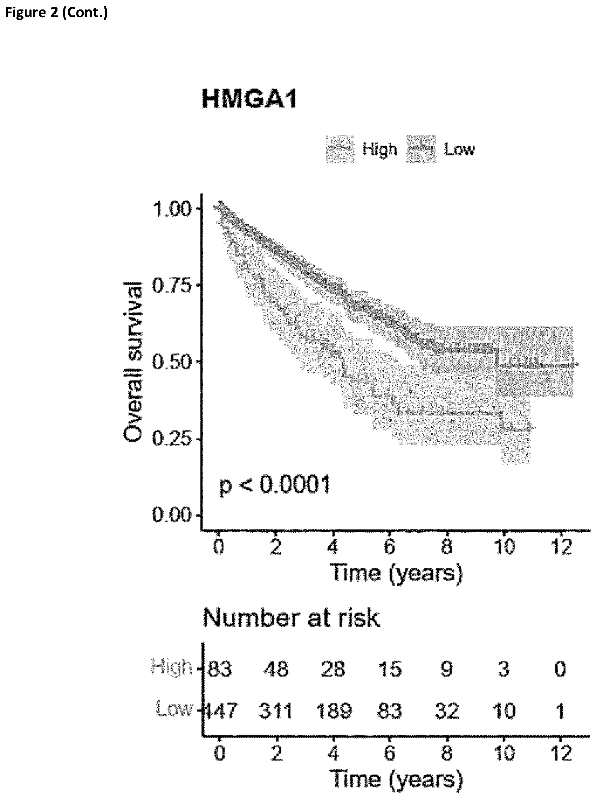

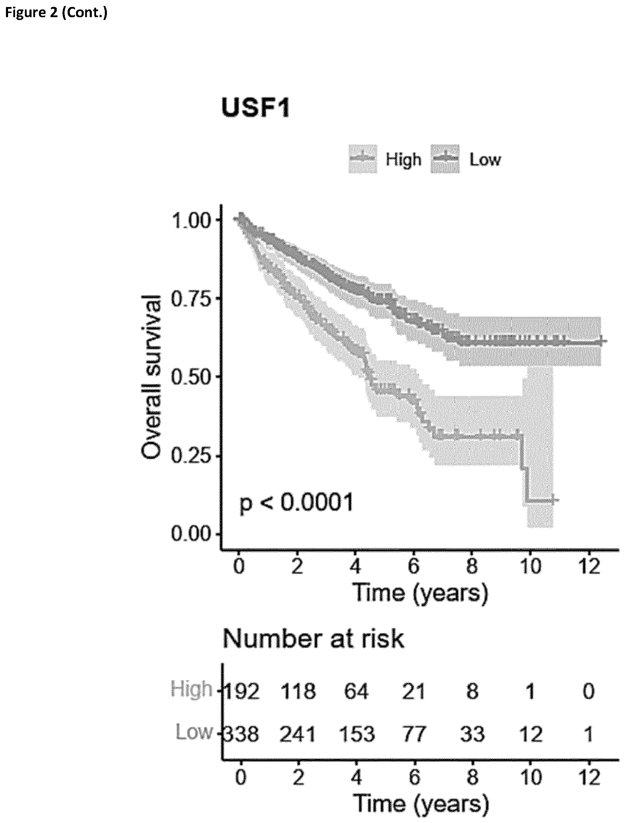

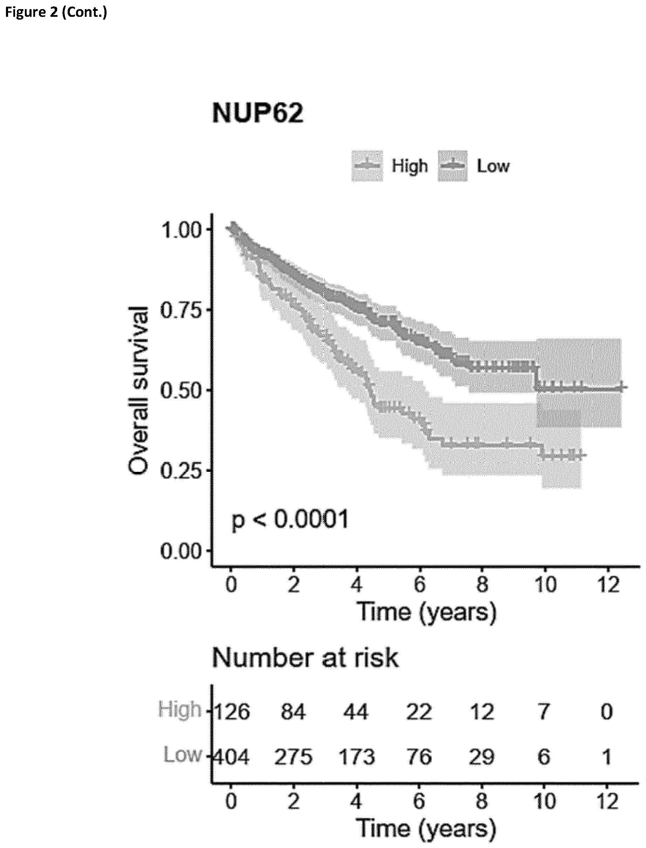

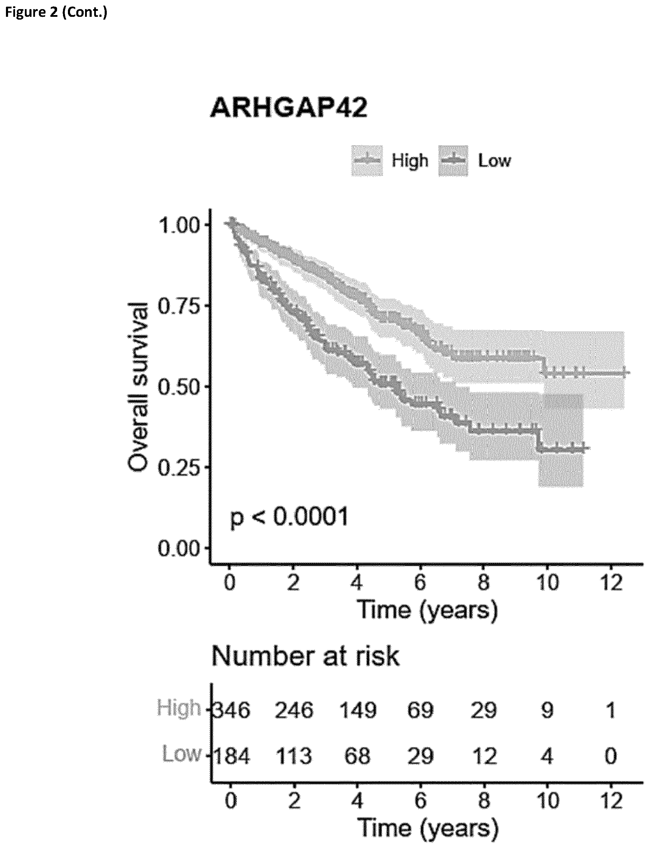

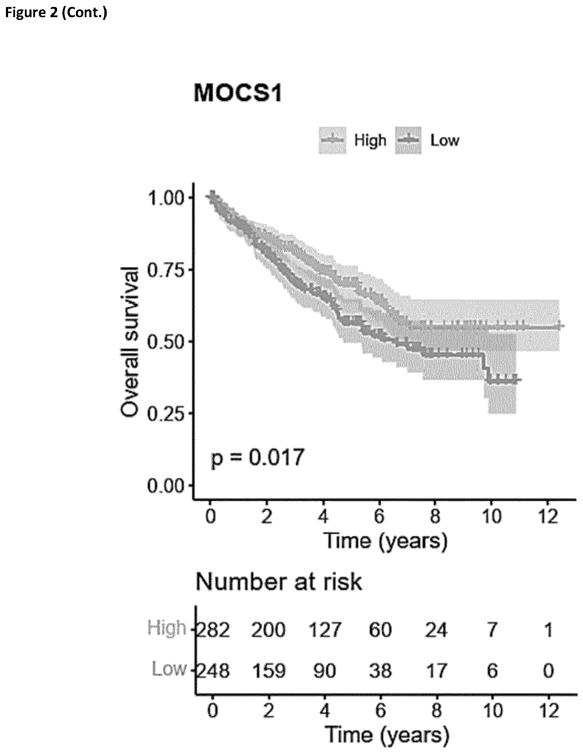

The predictive power of the 7 genes was tested in the TCGA KIRC cohort (*n* = 532) via the calculation of Log-Rank tests and the generation of Kaplan-Meier survival curves (**Figure 2**).

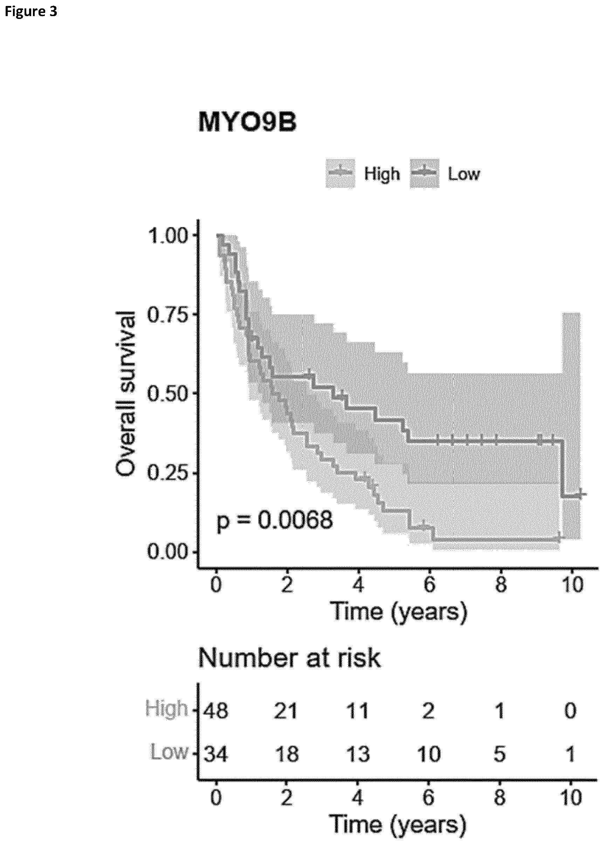

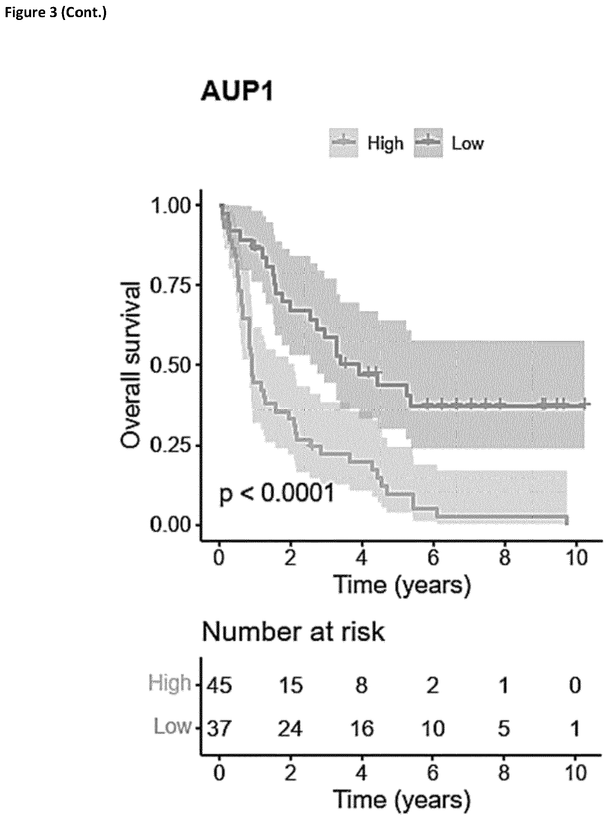

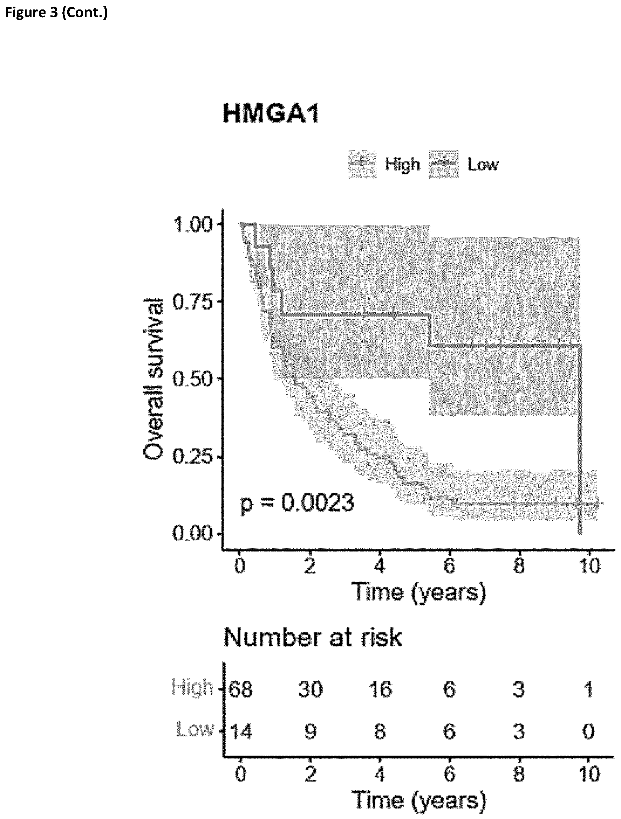

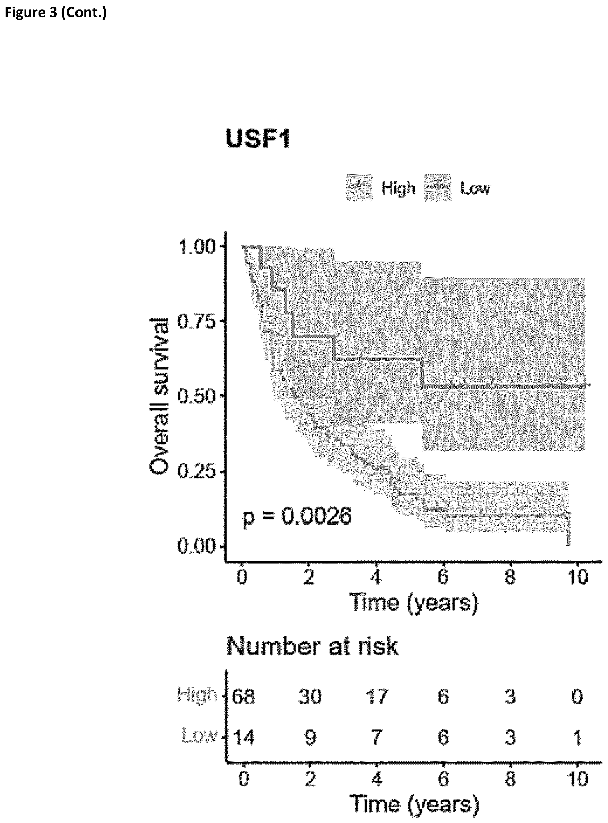

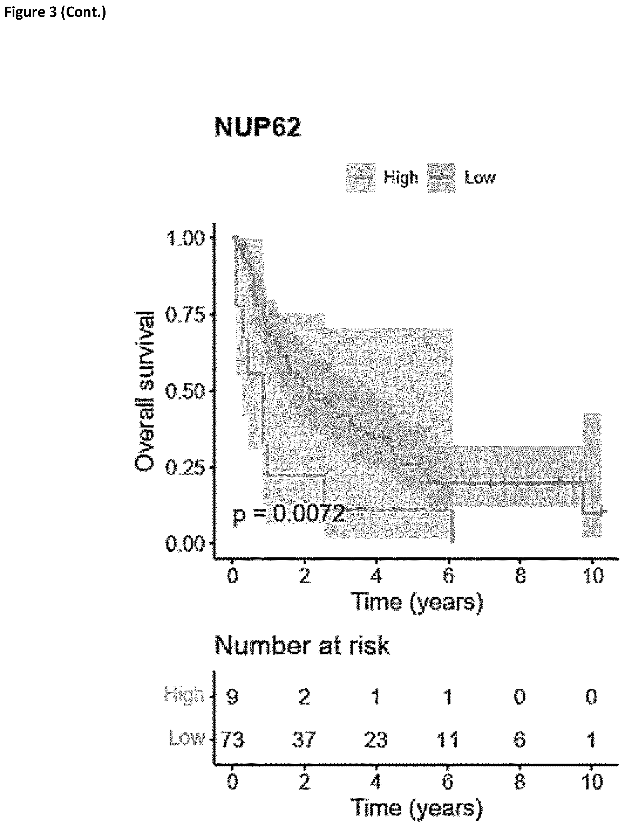

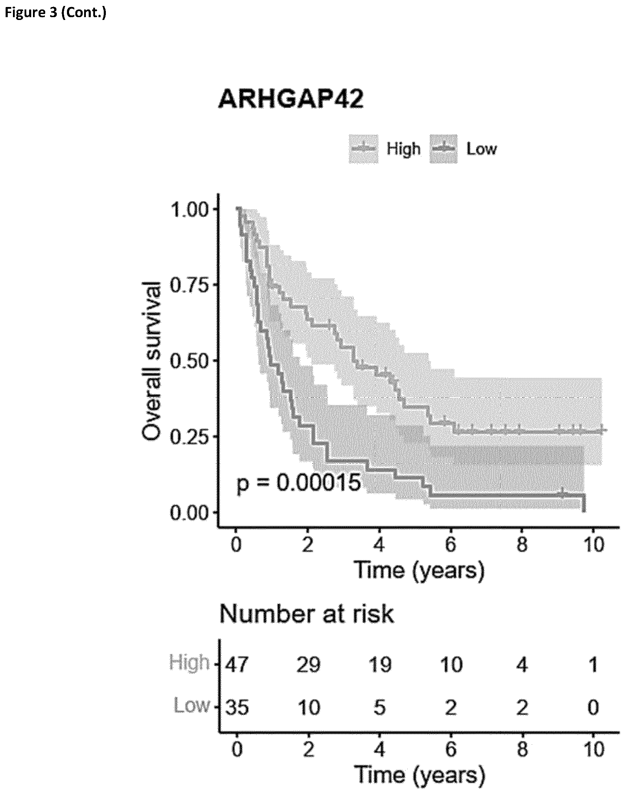

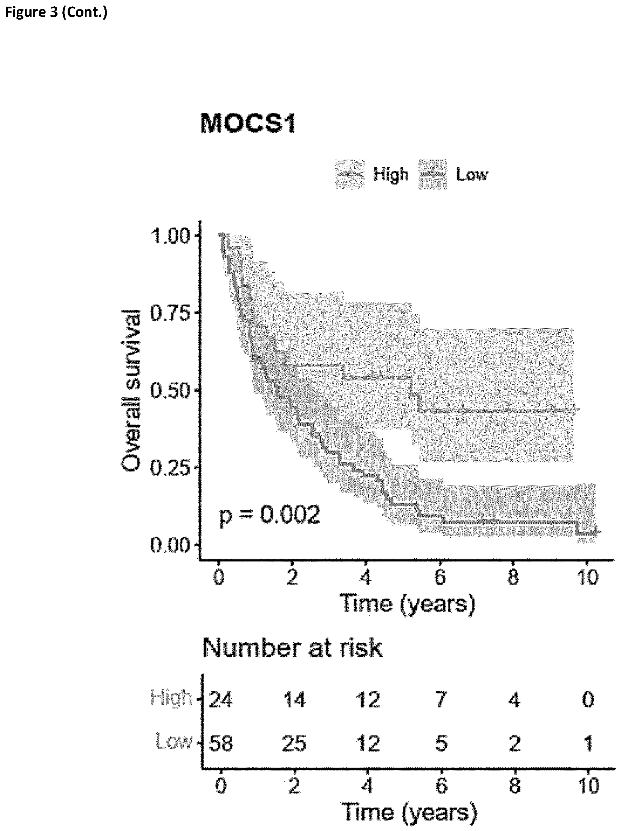

All selected genes showed a statistically significant (*P* < 0.05) effect for overall survival. The correlation between the expression level of each gene and the overall survival probability was in the same direction as previously described in the nivolumab-treated cohort. In addition, the predictive power of the selected genes was maintained when the cohort was reduced to stage IV patients only (**Figure 3**).

To confirm these findings, we generated univariate Cox PH models for each selected gene and calculated the HRs and the Wald test *P* values. All selected genes had a significant correlation with overall survival, with Wald test *P* < 0.05 in all cases (**Table 3**).

**Table 3. Genes with a statistically significant correlation with survival as observed in Cox PH univariate models in the TCGA KIRC cohort.**

| **Gene (high *vs* low)** | **Coefficient (β)** | **HR (95% CI)** | **Q (Wald test)** |
|---|---|---|---|
| MYO9B | 0.444 | 1.559 (1.139 - 2.133) | 0.005 ^{∗} |
| AUP1 | 1.089 | 2.973 (2.162 - 4.087) | <0.001 ^{∗} |
| HMGA1 | 0.724 | 2.064 (1.463 - 2.910) | <0.001 ^{∗} |
| USF1 | 0.876 | 2.402 (1.775 - 3.250) | <0.001 ^{∗} |
| NUP62 | 0.690 | 1.994 (1.462 - 2.721) | <0.001 ^{∗} |
| ARHGAP42 | -0.752 | 0.471 (0.349 - 0.636) | <0.001 ^{∗} |
| MOCS1 | -0.365 | 0.694 (0.514 - 0.938) | 0.017 ^{∗} |

| | | | |
|---|---|---|---|
| HR: Hazard Ratio. Asterisks indicate statistical significance (Q < 0.05). | | | |

The same analysis was restricted to stage IV patients, with consistent results (**Table 4**).

**Table 4. Genes with a statistically significant correlation with survival as observed in Cox PH univariate models in the stage IV patients from TCGA KIRC cohort.**

| **Gene (high *vs* low)** | **Coefficient (β)** | **HR (95% CI)** | **Q (Wald test)** |
|---|---|---|---|
| MYO9B | 0.717 | 2.048 (1.206 - 3.477) | 0.008 ^{∗} |
| AUP1 | 1.116 | 3.051 (1.811 - 5.141) | <0.001 ^{∗} |
| HMGA1 | 1.242 | 3.462 (1.841 - 8.090) | 0.004 ^{∗} |
| USF1 | 1.227 | 3.411 (1.463 - 7.956) | 0.005 ^{∗} |
| NUP62 | 0.936 | 2.550 (1.256 - 5.181) | 0.010 ^{∗} |
| ARHGAP42 | -0.921 | 0.398 (0.243 - 0.651) | <0.001 ^{∗} |
| MOCS1 | -0.938 | 0.392 (0.211 - 0.725) | 0.003 ^{∗} |

| | | | |
|---|---|---|---|
| HR: Hazard Ratio. Asterisks indicate statistical significance (Q < 0.05). | | | |

### Example. 2.5. Interaction between the selected genes and the therapeutic regimen in the CM-025 phase 3 trial

We tested the possible interaction between the expression level of the selected genes and the therapeutic regimen, either nivolumab or everolimus, among those patients from the randomised, phase 3 CM-025 trial. Cox PH models for the interaction between treatment and gene expression were generated for each gene expression category **(Table 5)**. Considering only this interaction, all models proved to have a significant predictive power, with at least one of the possible treatment and expression level combinations demonstrating a HR significantly different from 1 (*P* < 0.05, Wald test). In addition, all models for interaction had an LRT *P* < 0.01, demonstrating that the model with interaction has a significantly higher goodness-of-fit that the models with just the main effect of each variable separately.

**Table 5. Hazard Ratios and interaction term P values of each gene of interest with the treatment regime.**

| Gene | Expression level | HR (95% CI) | HR *P* | Interaction LRT *P* |
|---|---|---|---|---|
| MYO9B | High | 2.155 (0.916 - 5.072) | 0.079 | <0.001 ^{∗} |
| | Low | 0.586 (0.430 - 0.797) | <0.001 ^{∗} | |
| AUP1 | High | 0.796 (0.577 - 1.103) | 0.171 | <0.001 ^{∗} |
| | Low | 0.222 (0.106 - 0.464) | <0.001 ^{∗} | |
| HMGA1 | High | 0.690 (0.449 - 1.059) | 0.089 | <0.001 ^{∗} |
| | Low | 0.649 (0.439 - 0.960) | 0.030 ^{∗} | |
| USF1 | High | 0.589 (0.347 - 0.998) | 0.049 ^{∗} | <0.001 ^{∗} |
| | Low | 0.729 (0.518 - 1.027) | 0.071 | |
| NUP62 | High | 0.543 (0.339 - 0.868) | 0.011 ^{∗} | <0.001 ^{∗} |
| | Low | 0.753 (0.522 - 1.086) | 0.129 | |
| ARHGAP42 | High | 0.705 (0.505 - 0.985) | 0.040 ^{∗} | <0.001 ^{∗} |
| | Low | 0.437 (0.240 - 0.794) | 0.007 ^{∗} | |
| MOCS1 | High | 0.797 (0.541 - 1.174) | 0.250 | <0.001 ^{∗} |
| | Low | 0.579 (0.374 - 0.896) | 0.014 ^{∗} | |

| | | | | |
|---|---|---|---|---|
| HR: Hazard Ratio; CI: Confidence Interval; LRT: Likelihood Ratio Test. Asterisks indicate statistical significance (*P* < 0.05). | | | | |

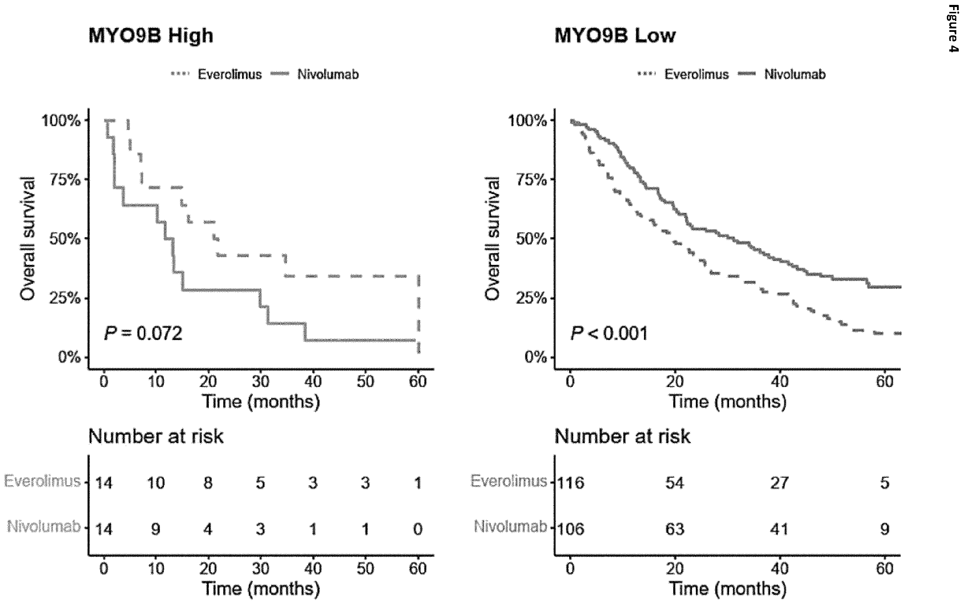

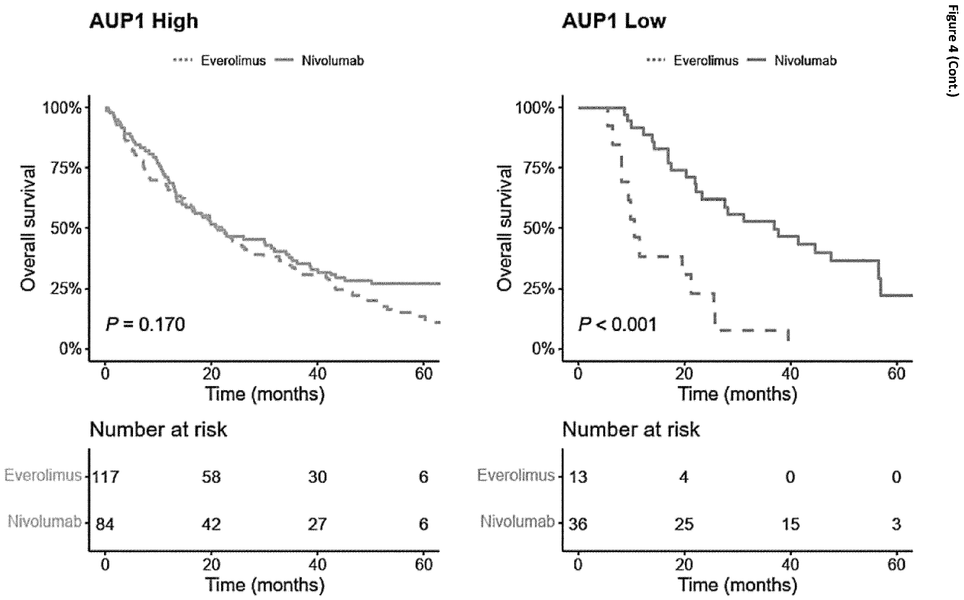

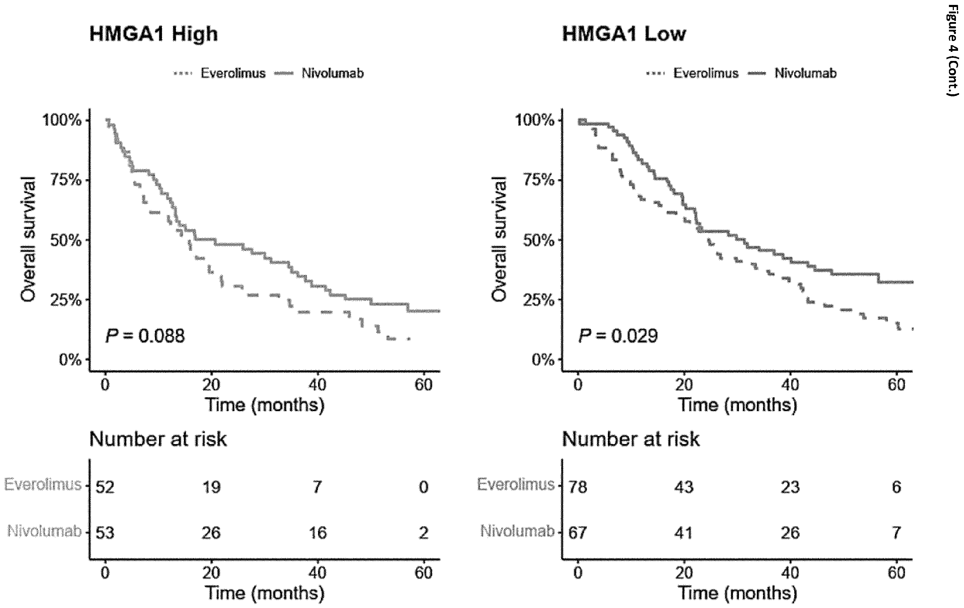

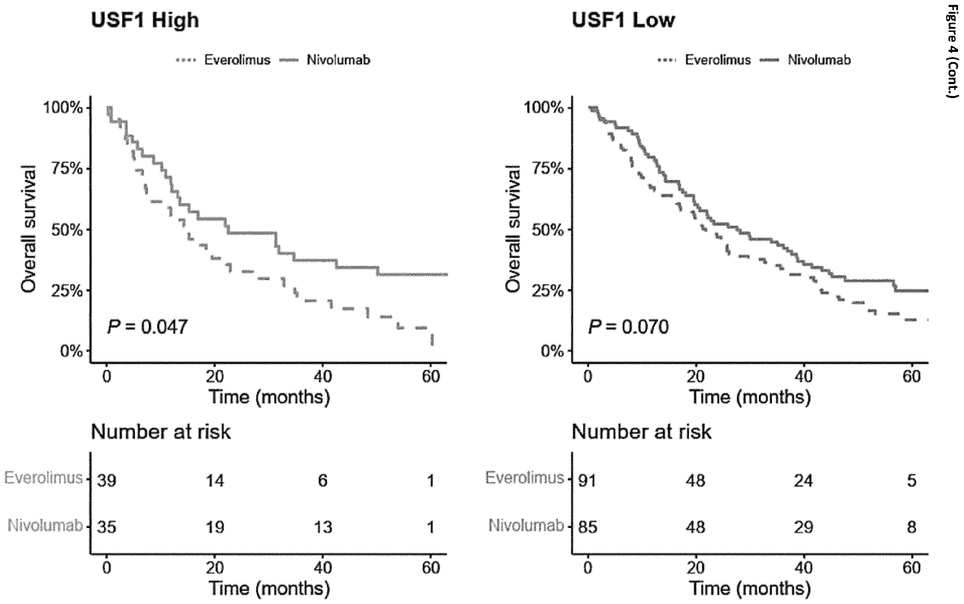

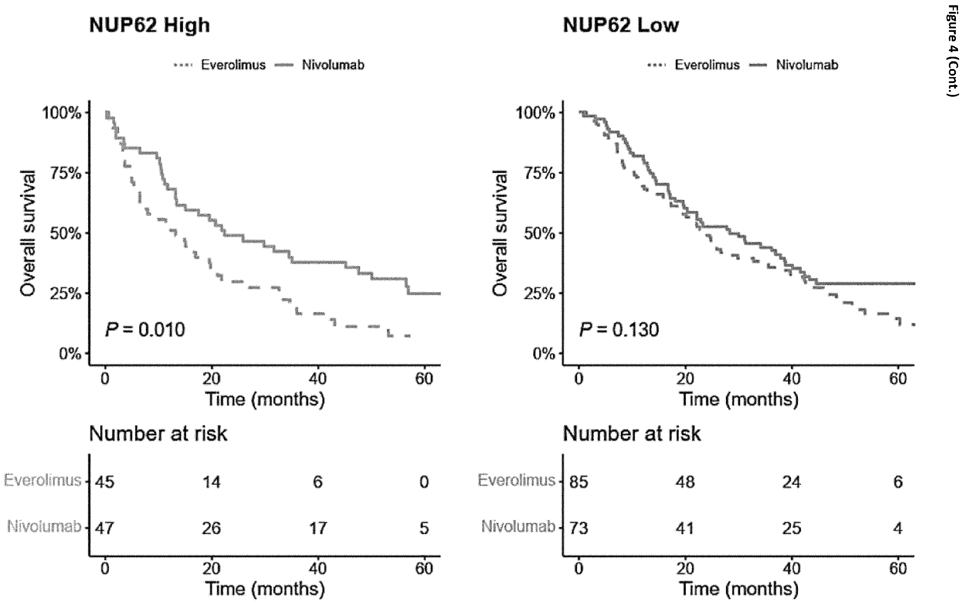

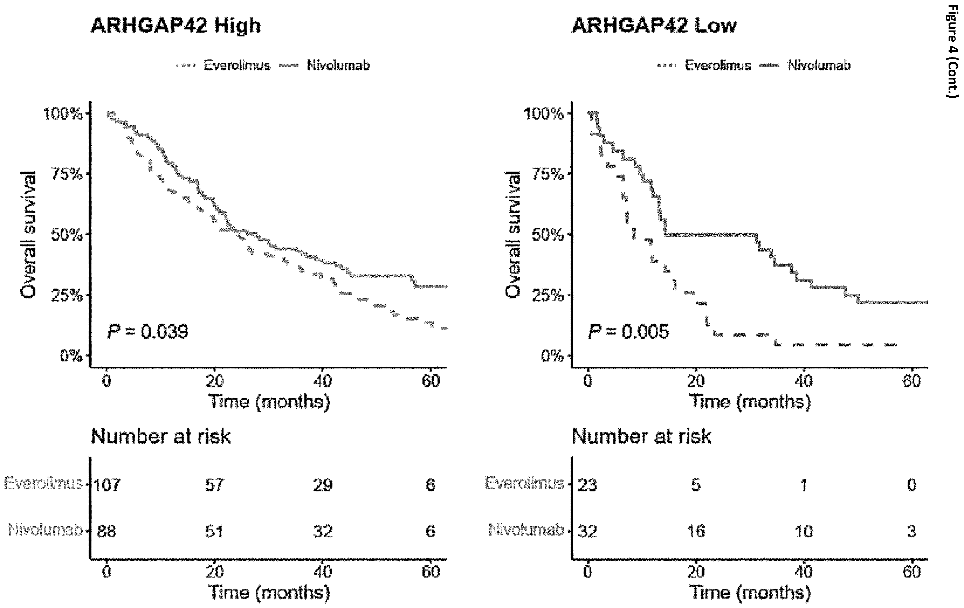

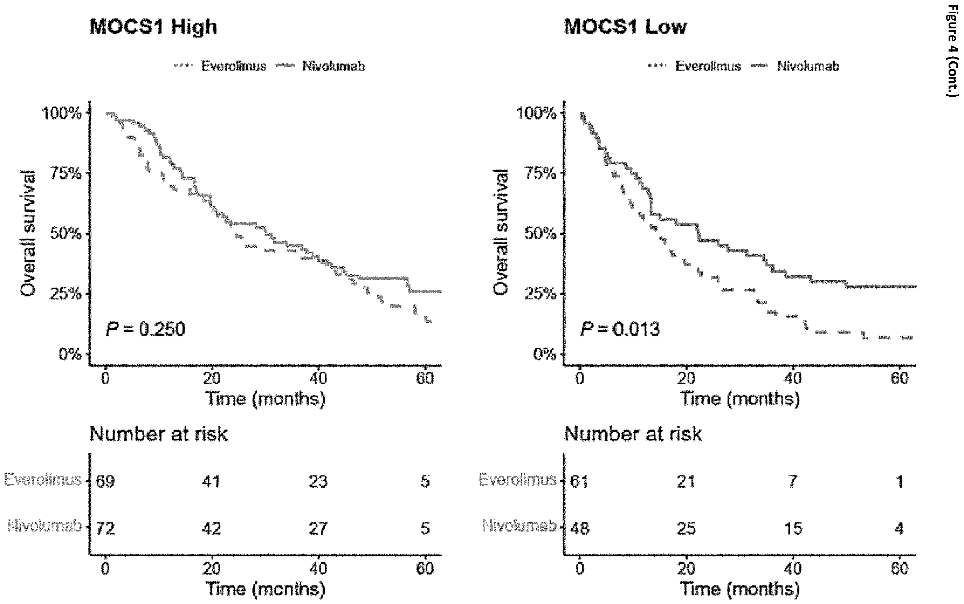

Kaplan-Meier survival curves and Log-Rank tests were generated comparing nivolumab *vs* everolimus for each gene expression level (**Figure 4**). Genes MYO9B, AUP1, HMGA1, and MOCS1 had a significant positive interaction with nivolumab when the expression was low, but the interaction was not significant when the expression was high. For genes USF1 and NUP62 the opposite was observed; they had a significant positive interaction with nivolumab when the expression was high, but not low. Regarding gene ARHGAP42, although the benefit from nivolumab is significant for each expression level, the differences in HR are wider when the expression level is low.

## Claims

1. *In vitro* method for predicting the prognosis of renal carcinoma in a patient which comprises:
a. Assessing the level of expression of at least one of the following genes included in the group consisting of: MYO9B, AUP1, HMGA1, USF1, NUP62, ARHGAP42 or MOCS1, in a biological sample obtained from the patient,
b. Wherein the identification of a higher level of expression of MYO9B, AUP1, HMGA1, USF1 or NUP62 which respect to a pre-established threshold value is indicative of poor prognosis; or wherein the identification of a lower level of expression of MYO9B, AUP1, HMGA1, USF1 or NUP62 which respect to a pre-established threshold value is indicative of good prognosis; or
c. Wherein the identification of a higher level of expression of ARHGAP42 or MOCS1 which respect to a pre-established threshold value is indicative of good prognosis; or wherein the identification of a lower level of expression of ARHGAP42 or MOCS1 which respect to a pre-established threshold value is indicative of poor prognosis.

2. *In vitro* method for predicting the benefit of patients suffering from renal carcinoma from a treatment with antibody-based immunotherapy which comprises:
a. Assessing the level of expression of at least one of the following genes included in the group consisting of: MYO9B, AUP1, HMGA1, USF1, NUP62, ARHGAP42 or MOCS1, in a biological sample obtained from the patient,
b. Wherein the identification of a lower level of expression of MYO9B, AUP1, HMGA1, ARHGAP42, or MOCS1 which respect to a pre-established threshold value is indicative that the patient is benefitting from the therapy; or
c. Wherein the identification of a higher level of expression of USF1 or NUP62 which respect to a pre-established threshold value is indicative that the patient is benefitting from the therapy.

3. *In vitro* method for deciding or recommending whether to treat patients suffering from renal carcinoma with antibody-based immunotherapy which comprises:
a. Assessing the level of expression of at least one of the following genes included in the group consisting of: MYO9B, AUP1, HMGA1, USF1, NUP62, ARHGAP42, or MOCS1 in a biological sample obtained from the patient,
b. Wherein if a lower level of expression of MYO9B, AUP1, HMGA1, ARHGAP42 or MOCS1 is identified which respect to a pre-established threshold value a treatment with antibody-based immunotherapy is recommended; or
c. Wherein if a higher level of expression of USF1 or NUP62 is identified which respect to a pre-established threshold value a treatment with antibody-based immunotherapy is recommended.

4. *In vitro* method, according to any of the claims 2 or 3, wherein the antibody-based immunotherapy comprises an anti-PD-1 or an anti-PD-Ll antibody.

5. *In vitro* method, according to any of the claims 2 to 4, wherein the antibody-based immunotherapy comprises an anti-PD-1 or an anti-PD-Ll antibody selected from: nivolumab, pembrolizumab, dostarlimab, sintilimab, cemiplimab, atezolizumab, durvalumab or avelumab.

6. *In vitro* use of at least one of the following genes included in the group consisting of: MYO9B, AUP1, HMGA1, USF1, NUP62, ARHGAP42 or MOCS1, for predicting the prognosis of renal carcinoma or for predicting the benefit of patients suffering from renal carcinoma from a treatment with antibody-based immunotherapy.

7. *In vitro* use of at least one of the following genes included in the group consisting of: MYO9B, AUP1, HMGA1, USF1, NUP62, ARHGAP42 or MOCS1, according to claim 6, for predicting the benefit of patients suffering from renal carcinoma from a treatment with antibody-based immunotherapy comprising an anti-PD-1 antibody.

8. *In vitro* use of at least one of the following genes included in the group consisting of: MYO9B, AUP1, HMGA1, USF1, NUP62, ARHGAP42 or MOCS1, according to any of the claims 6 or 7, for predicting the benefit of patients suffering from renal carcinoma from a treatment with antibody-based immunotherapy comprising an anti-PD-1 or an anti-PD-L1 antibody selected from: nivolumab, pembrolizumab, dostarlimab, sintilimab, cemiplimab, atezolizumab, durvalumab or avelumab.

9. Kit suitable for performing the method of any of the claims 1 to 5 which consist of reagents or tools to assess the level of expression of the genes: MYO9B, AUP1, HMGA1, USF1, NUP62, ARHGAP42 and MOCS1.

10. Anti-PD-1 or anti-PD-L1 antibody for use in the treatment of renal carcinoma in a patient, wherein the patient is **characterized by** a lower expression level of at least one of the following genes included in the group consisting of: MYO9B, AUP1, HMGA1, ARHGAP42, or MOCS1; or by a higher level of expression level of at least one of the following genes included in the group consisting of: USF1 or NUP62, which respect to a pre-established threshold value.

11. Anti-PD-1 or anti-PD-L1 antibody for use in the treatment of renal carcinoma in a patient, according to claim 10, wherein the patient is **characterized by** a lower expression level of at least one of the following genes included in the group consisting of: MYO9B, AUP1, HMGA1, ARHGAP42, or MOCS1; or by a higher level of expression of at least one of the following genes included in the group consisting of: USF1 or NUP62, which respect to a pre-established threshold value, and wherein the method comprises the step of determining whether the patient has a lower expression level of at least one of the following genes included in the group consisting of: MYO9B, AUP1, HMGA1, ARHGAP42, or MOCS1, or a higher level expression level of at least one of the following genes included in the group consisting of: USF1 or NUP62, which respect to a pre-established threshold value.

12. Anti-PD-1 or anti-PD-L1 antibody for use, according to any of the claims 10 or 11, selected from: nivolumab, pembrolizumab, dostarlimab, sintilimab, cemiplimab, atezolizumab, durvalumab or avelumab.
